# EUROPÄISCHE PATENTSCHRIFT

(11) **EP 0 811 397 B1**
(45) Veröffentlichungstag und Bekanntmachung des Hinweises auf die Patenterteilung: **23.03.2005**
(21) Anmeldenummer: 97109044.4
(22) Anmeldetag: 04.06.1997
(51) Int. Cl.: A61N 1/375, H01R 13/52

(54) **Implantierbare Einheit**
Implantable unit
Unité implantable

(30) Priorität: 05.06.1996 DE 19622669
(43) Veröffentlichungstag der Anmeldung: 10.12.1997
(73) Patentinhaber: Cochlear Limited, Lane Cove, NSW 2066 (AU)
(72) Erfinder: Volz, Andreas, Dipl.-Ing. (FH), 81927 München (DE); Leysieffer, Hans, Dr. Dipl.-Ing., 82024 Taufkirchen (DE)
(74) Vertreter: Schwan, Gerhard, Dipl.-Ing.

(56) Entgegenhaltungen:
- GB-A- 2 134 335
- US-A- 4 495 917
- US-A- 5 046 242
- US-A- 5 336 246

## Beschreibung

Die Erfindung bezieht sich auf eine implantierbare Einheit mit mindestens einer Kontaktanordnung zur Verbindung einer in einem Gehäuse hermetisch dicht untergebrachten elektrischen oder elektronischen Vorrichtung mit mindestens einem aus dem Gehäuse herausgeführten Anschlußkabel. Insbesondere befaßt sich die Erfindung mit einer lösbaren elektrischen Verbindung zwischen einem Implantatgehäuse, das aktive elektronische Komponenten beherbergt, und Sensoren oder Aktoren, die an einen ganz gezielten Ort im Körper zu plazieren sind und von den aktiven elektronischen Komponenten betrieben werden.

Derartige implantierbare Steckverbindungen werden bei Herzschrittmachern, Defibrillatoren und Kardiovertern eingesetzt. Beim Austausch der implantierten Elektronik, die auf Grund der verbrauchten Energiequelle erforderlich wird, wird die operationstechnisch aufwendig zu ex- und implantierende Sensorik und Aktorik nach Möglichkeit vor Ort belassen. Dies hat bei Herzschrittmachern zu einem Standard geführt, so daß unterschiedliche Modelle an die einmal gelegte Sensorik und Aktorik angeschlossen werden können. Anforderungen an den Elektrodenstecker und die Steckerbuchse sind in der DIN VDE 0750 Teil 91 festgelegt, die auch eine genaue Vorgabe der Maße und Toleranzen beinhaltet. Gemäß dieser DIN-Norm ausgebildete Stecker funktionieren ähnlich wie ein Bananenstecker, verfügen jedoch über zwei Kontaktflächen mit unterschiedlichem Durchmesser, die durch dichtende O-Ringe (Dichtlippen) voneinander getrennt sind. Die Steckeraufnahme ist entsprechend dem Stecker ebenfalls in dieser DIN-Norm maßlich festgelegt. Die Durchmesser der Dichtlippen und der Steckeraufnahme bestimmen den Anpreßdruck und die hiermit erzielbare Dichtwirkung gegen eindringende Körperflüssigkeit. Die Norm beinhaltet weiterhin eine Prüfvorschrift bezüglich der Isolationsimpedanz, die größer als 50 kΩ sein muß.

Zahlreiche Patentschriften befassen sich mit der produktionstechnischen Ausführung der Steckeraufnahme an einem hermetisch dichten Gehäuse, der produktionstechnischen Ausführung des Steckers, der Art der Kontaktgebung und der Fixation des Steckers im Steckergehäuse. Alle der folgenden Patentschriften haben eine zylinderförmige Öffnung zur Aufnahme eines zylindrischen Steckers mit einer oder mehreren Kontaktstellen und isolierenden Dichtlippen gemeinsam:
EP-A-0 052 690, US-A-4 262 673, EP-A-0 006 281, EP-B-0 052 879, EP-A-0 442 807, WO-A-90/02581, WO-A-91/04069, WO-A-91/16947, WO-A-93/05844, WO-A-93/02742, WO-A-89/05170, EP-A-0 357 941.

EP-A-0 587 379 und EP-A-0 306 443 beziehen sich auf eine koaxiale Ausführungsmöglichkeit des beschriebenen Steckverbindungssystems.

Die Druckschrift EP-A-0 339 877 sieht die Kontaktgebung zum zylindrischen Stecker durch Verwendung von leitfähigem Silikon in der Steckeraufnahme vor. Durch Wechsel von leitfähigen und nicht leitfähigen Schichten können mehrere Kontakte auf dem zylindrischen Stecker realisiert werden. Da sowohl die Kontakte als auch die Isolatoren flexibel sind, kann auf die Ausbildung von Dichtlippen verzichtet werden, indem durch Anpassung der Durchmesser von Steckeraufnahme und Stift ein entsprechender Anpreßdruck sowohl in der Kontaktzone als auch in der isolierenden Zone realisiert wird. Der Anpreßdruck ist durch den Gleitweg limitiert, der beim Einführen der Stecker handhabbar durchfahren werden muß.

Die deutsche Offenlegungsschrift DE-A-33 31 620 weicht von dem zylindrischen Stecker, der mehrere Kontaktflächen beinhalten kann, die durch Dichtlippen voneinander isoliert werden, ab und sieht mehrere Kontaktstifte vor, die senkrecht aus einer Steckergrundplatte herausstehen. Um die Kontakte gelegte O-Ringe sorgen für die Abdichtung, wenn die Steckergrundplatte gegen das Gehäuse geschraubt wird. Durch Verwendung der Schrauben zur Erzeugung der Dichtwirkung kann ein wesentlich höherer Anpreßdruck erzielt werden, da dieser Druck nicht beim manuellen Einbringen der Stecker überwunden werden muß. Der Erfinder gibt an, einen gasdichten Verschluß zu erzielen. Diese Druckschrift kommt der nachfolgend beschriebenen Erfindung am nächsten, die ebenfalls höhere Ansprüche an die Isolation zwischen den Kontakten stellt.

Die Druckschrift EP-A-0 001 897 beschreibt die elektrische Verbindungsmöglichkeit zwischen zwei Substraten unter der Verwendung von wechselnden Schichten von nicht leitfahigen und leitfähigen Silikonen. Es wird davon ausgegangen, daß sich die Substrate direkt im Körpermedium befinden.

Implantate der eingangs genannten Art, z.B. implantierbare Hörgeräte, Herzschrittmacher, Medikamentenpumpen etc., sollten bei der Implantation in den Körper nach Möglichkeit wenig Platz erfordern und somit weitgehend miniaturisiert werden. Werden im Zuge einer solchen Miniaturisierung die aus dem Stand der Technik bekannten Steckverbindungen ebenfalls miniaturisiert, so erhält man Steckverbindungen mit sehr dünnen, länglichen Kontakten, die beim Einstecken in entsprechende Buchsen leicht abgebrochen, verbogen oder sonstwie beschädigt werden können.

Es ist daher eine Aufgabe der vorliegenden Erfindung, eine implantierbare Einheit der eingangs genannten Art zu schaffen, bei der sich einerseits unter Vermeidung der Verwendung von übermäßig empfindlichen Bauteilen ein hoher Miniaturisierungsgrad erreichen läßt, und bei denen andererseits für einen sicheren und zuverlässigen Kontakt zwischen der in dem Gehäuse untergebrachten Vorrichtung und dem bzw. den Anschlußkabeln gesorgt ist.

Diese Aufgabe wird gemäß der vorliegenden Erfindung gelöst, die in Anspruch 1 definiert ist.

Das Dokument GB-A-2134335 offenbart eine Vorrichtung gemäß Oberbegriff des Anspruchs 1.

Gemäß einer Ausführungsform der Erfindung weist der Verschlußmechanismus eine mit dem mindestens einen Anschlußkabel verbundene Verschlußkappe auf, in welcher mindestens ein erster Kontakt und/oder mindestens ein zweiter Kontakt angeordnet ist, wobei eine Eingriffsanordnung vorgesehen ist, um die Verschlußkappe mit dem Gehäuse in Eingriff halten. In diesem Fall bildet die Verschlußkappe mit den darin befindlichen Kontakten eine Einheit, die z.B. über ein dem Gehäuse zugeordnetes Gewinde und eine der Verschlußkappe zugeordnete Schraube an dem Gehäuse befestigt werden kann. Alternativ kann eine zwischen der Verschlußkappe und dem Gehäuse wirkende Rastverbindung vorgesehen sein.

Soll ein Austausch nur des Anschlußkabels möglich sein, so ist es von Vorteil, wenn das Anschlußkabel mit einem Anschlußstück versehen ist, das einen oder mehrere erste oder zweite Kontakte trägt, und wenn der Verschlußmechanismus eine Verschlußkappe mit einer Aufnahme für das Anschlußstück aufweist. Die Möglichkeit des Austausches des Anschlußkabels ist insbesondere dann von Vorteil, wenn mit der in dem Gehäuse untergebrachten Vorrichtung mehrere Anschlußkabel zu verbinden sind. Auch bei dieser Ausführungsform dienen eine Schraube und ein zugehöriges Gewinde oder eine Rastverbindung dazu, die Verschlußkappe mit dem Gehäuse in Eingriff zu halten.

Wenn für den Eingriff zwischen Verschlußkappe und Gehäuse eine Schraube benutzt wird, ist die Anordnung vorzugsweise so getroffen, daß für einen Kontakt zwischen den ersten und zweiten Kontakten ein definiertes Drehmoment, welches auf die Schraube aufgebracht wird, erforderlich ist. Durch Verwendung eines geeigneten Werkzeuges, insbesondere eines Drehmomentschlüssels, wird somit eine übermäßige Beanspruchung sowohl der Schraube als auch der miteinander in Kontakt tretenden Bauteile vermieden, und dennoch kann sich der Operateur sicher sein, daß die gewünschten Kontakte hergestellt werden. Desweiteren kann die Verschlußkraft somit auch bei einer hohen Anzahl von zu schließenden Dichtungen und Kontakten sicher eingeleitet werden.

Je nach Art der zu implantierenden Vorrichtung kann es von Vorteil sein, wenn das Einbringen der Anschlußstücke in einer Richtung senkrecht zu den Anschlußkabeln erfolgt und die Kraft zum Verbinden der Kontakte in einer Richtung senkrecht zu der Wirkungsrichtung dieser Kraft aufgebracht werden kann. Zu diesem Zweck kann in weiterer Ausgestaltung der Erfindung an dem mindestens einen Anschlußkabel ein Anschlußstück vorgesehen sein und das Gehäuse eine wannenförmige Verlängerung zur Aufnahme des mindestens einen Anschlußstücks aufweisen, wobei ferner ein Keil zum Einbringen zwischen die dem Gehäuse entfernt liegende Wandseite der wannenförmigen Verlängerung und ein in die wannenförmige Verlängerung eingelegtes Anschlußstück vorgesehen ist. Wird der Keil beispielsweise mittels einer eigens dafür vorgesehenen Schraube eingebracht und fixiert, preßt er das Anschlußstück in einer Richtung senkrecht zur Schraubenachse gegen das Gehäuse an

Durch diese Weg-Kraft Transformation ist der Verschlußmechanismus auch bei einem parallel zur Hautfläche des Patienten implantierten Gehäuse, wie z.B. einem im Schädelknochen eingebetteten Gehäuse, von oben bedienbar, so daß zum Auswechseln bzw. Ankoppeln der Anschlußkabel das Gehäuse nicht aus seiner festen Verankerung, wie z.B. einem Knochenbett, entfernt werden muß. Somit hat der behandelnde Chirurg beide Hände frei für den Anschließvorgang, und er muß nicht wie bei konventionellen Anordnungen das Gehäuse für den Verbindungsvorgang halten. Die Gefahr, während des Anschließens auf die Leitungen, die mit der Aktorik oder Sensorik verbunden sind, Zug auszuüben, was zu einer Beschädigung der im Bedarfsfall sehr dünnen Leitungen (insbesondere bei mehrkanaligen Systemen) oder der Sensorik und Aktorik führen kann, wird somit erheblich gemindert. Bei vielen herkömmlichen Vorrichtungen, bei denen zuerst der Anschlußvorgang abgeschlossen werden muß, bevor sie am endgültigen Implantationsort plaziert werden, ist außerdem für den Anschlußvorgang eine bestimmte Länge an überschüssigem Kabel erforderlich, das am Implantationsort in eine Schleife um das Gehäuse gelegt wird. Im Gegensatz dazu ermöglicht das hier vorgeschlagene Ankoppelprinzip direkte und kurze Leitungswege zwischen der in dem Gehäuse untergebrachten Vorrichtung und den daran anzuschließenden sensorischen oder aktorischen Komponenten.

Zum Fixieren des Keils kann jedoch auch zwischen dem Keil und dem Gehäuse eine Rastvorrichtung vorgesehen sein, so daß der Keil manuell eingebracht und auf ein Werkzeug zum Bedienen der Schraube verzichtet werden kann.

Bevorzugte Ausführungsformen der Erfindung werden im folgenden unter Bezugnahme auf die beiliegenden Zeichnungen näher erläutert. Es zeigen:
- FIG. 1: eine Schnittansicht eines implantierbaren Gehäuses;
- FIG. 2: eine schematische perspektivische Ansicht auf die Stirnseite des in FIG. 1 gezeigten Gehäuses;
- FIG. 3: eine Schnittansicht die einen Teil eines implantierbaren Gehäuses ähnlich FIG. 1 zeigt;
- FIG. 4: eine Ansicht ähnlich FIG. 3 einer abgewandelten Ausführungsform des in FIG. 1 gezeigten Gehäuses
- FIG. 5: eine Schnittansicht einer Kontaktanordnung in geschlossenem Zustand und in großerem Maßstab;
- FIG. 6: eine Schnittansicht ähnlich FIG. 5 der Kontaktanordnung in geöffnetem Zustand;
- FIG. 7: eine perspektivische Ansicht eines implantierbaren Gehäuses gemäß einer weiteren Ausführungsform; und
- FIG. 8: eine perspektivische Explosionszeichnung des FIG. 7 gezeigten implantierbaren Gehäuses sowie der zum Fixieren der Kontakte und zum Verschließen des Gehäuses verwendeten Bauteile.

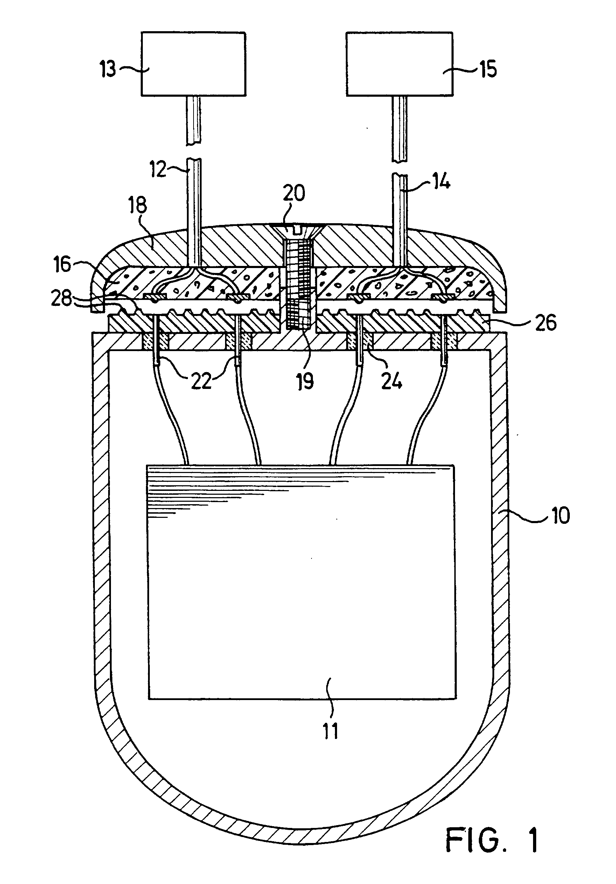

FIG. 1 zeigt einen Schnitt durch eine implantierbare Einheit mit einem Gehäuse 10 zur Aufnahme einer als Block angedeuteten elektrischen oder elektronischen Vorrichtung 11, beispielsweise dem Signalwandler eines implantierbaren Hörgeräts, der Signalverarbeitungselektronik eines Herzschrittmachers etc. Die Verbindung der in dem hermetisch dichten Gehäuse 10 untergebrachten Vorrichtung mit gleichfalls nur als Block dargestellten aktorischen oder sensorischen Komponenten 13, 15 erfolgt über Anschlußkabel 12, 14, deren Enden in Anschlußstücke 16 eingebettet sind, die bei der Implantation der Einheit in eine an der Stirnseite des Gehäuses 10 vorgesehene Endkappe 18 eingelegt werden. Dem Fixieren der Anschlußstücke 16 dient eine Schraubverbindung, die von einem dem Gehäuse 10 zugeordneten Gewinde 19 und einer der Verschlußkappe 18 zugeordneten Schraube 20 gebildet wird. FIG. 1 zeigt eine Ausführungsform für eine implantierbare Einheit mit einem sensorischen und einem aktorischen Element 13 bzw. 15, die räumlich getrennt voneinander anzuordnen sind, und die daher eigener Anschlußkabel bedürfen. Es versteht sich jedoch, daß sich das im folgenden beschriebene Prinzip in gleicher Weise in Ausführungsformen mit nur einem oder mit mehr als zwei Anschlußkabeln verwirklichen läßt. Führen Sensor- und Aktorkanäle zu unterschiedlichen Implantationsorten, so sollte pro Implantationsort ein Anschlußkabel existieren, dessen Anschlußstück unabhängig von den anderen gelöst werden kann, so daß ein Fehler in der Sensorik oder Aktorik nicht eine komplette erneute Operation (RE-OP) erforderlich macht.

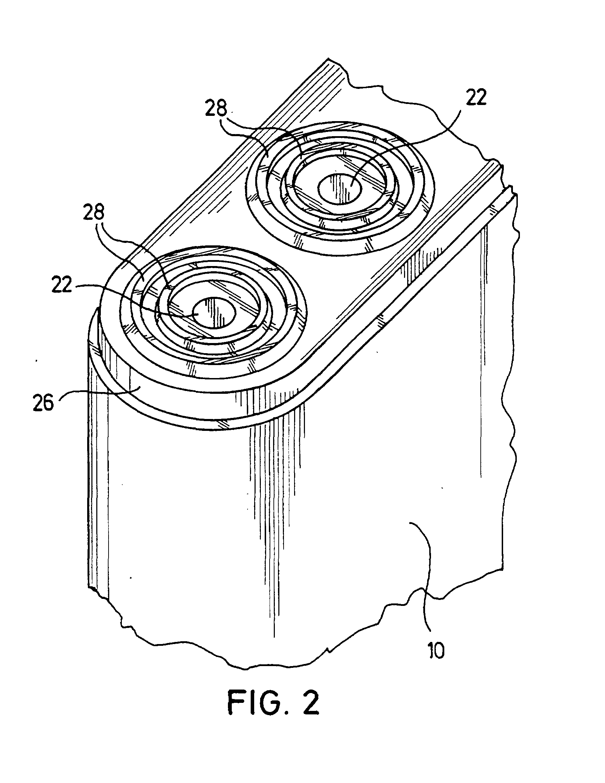

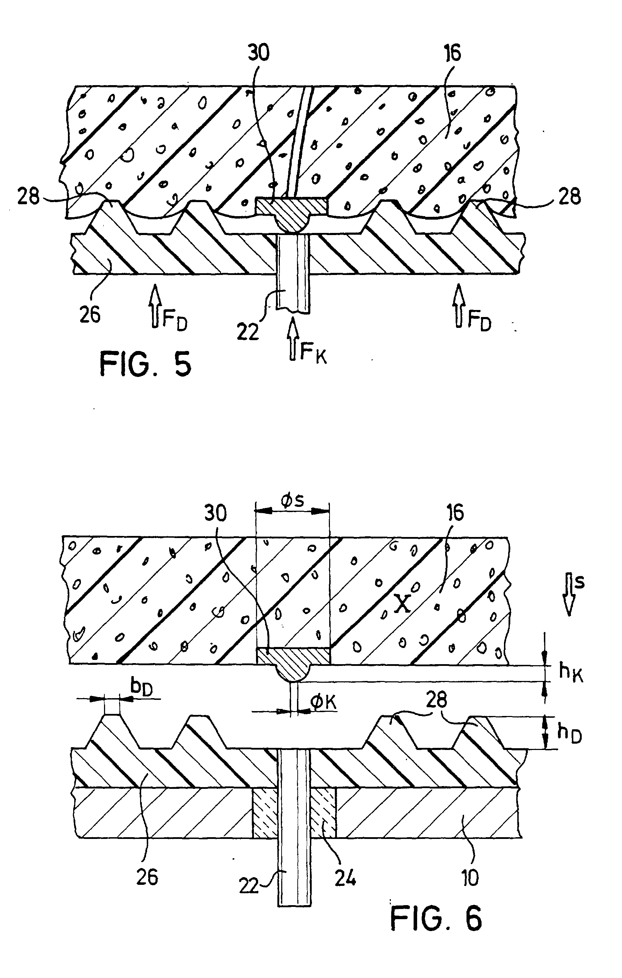

Zur Ausbildung der gehäuseseitigen Kontakte sind an der Stirnseite des Gehäuses 10 Durchführungen 22 mit flacher Stirnseite vorgesehen, die durch einen Isolator 24, wie z.B. Keramik, von dem in der Regel aus Metall gefertigten Körper des Gehäuses getrennt sind und starr gehalten werden. Der Isolator 24 sorgt ferner für eine hermetische Abdichtung zwischen den Durchführungen 22 und dem Gehäuse 10. Auf der die drahtförmigen Durchführungen 22 enthaltenden Seite des Gehäuses 10 ist ein Formkörper 26 aus einem elektrisch isolierenden, unelastischen Werkstoff, z.B. Polycarbonat, angebracht, dessen Oberfläche mit den Stirnflächen der Durchführungsdrähte 22 in einer Ebene abschließt. Um die aus den Stirnfläche der Durchführungsdrähte 22 bestehenden Kontaktflächen gegen Körperflüssigkeit abzudichten, sind an dem Formkörper 26 ein, zwei oder mehrere aus der Ebene herausragende Dichtungsstege 28 vorgesehen, die sich beim Verschrauben von Gehäuse 10 und Endkappe 18 in die aus einem elastischen Werkstoff gefertigten Anschlußstücke 16 eingraben. Bei der in FIG. 1 und insbesondere in FIG. 2 gezeigten Ausführungsform, sind für jede der Durchführungen 22 zwei kreisförmige Dichtungsstege 28 vorgesehen, welche die jeweilige Durchführung konzentrisch umgeben. Statt dessen oder zusätzlich können auch Dichtungsstege vorgesehen werden, welche mehrere Kontakte, z.B. jeweils die einem Anschlußstück zugeordneten Kontakte oder etwa alle Kontakte der gesamten Einheit, umschließen. Die Abmessungen der Formkörper 26, der Anschlußstücke 16 sowie der Endkappe 18 sind dabei so gewählt, daß der elastische Werkstoff der Anschlußstücke 16, bei dem es sich vorzugsweise um Silikon handelt, beim Aufschrauben der Endkappe 18 nicht über seine Elastizitätsgrenze hinaus beansprucht wird, so daß eine Rückstellkraft erhalten bleibt, die einen hohen Druck auf den Dichtungsstegen erzeugt und die in FIG. 5 als Dichtungskraft ***F***_{***D***} bezeichnet ist. Auf gleiche Weise wird beim Aufschrauben der Endkappe 18 zwischen den Durchführungen 22 und den kappenseitigen Kontakten 30, die in dem elastischen Werkstoff der Anschlußstücke 16 eingebettet sind, eine Kontaktkraft ***F***_{***K***} erzeugt, indem die Kontakte 30 beim Auftreffen auf die nicht zurückweichende Kontaktflächen der Durchführungen 22 in den elastischen Werkstoff der Anschlußstücke 16 geschoben werden, der wie eine Feder diese Kontaktkraft aufrechterhält. Die Kontaktkraft ***F***_{***K***} wird vorzugsweise so groß gewählt, daß die Oberflächen der metallischen Kontakte zu fließen beginnen und einen gasdichten Verschluß der Kontaktstelle gewährleisten. Vorzugsweise ist in der für die Kontakte 30 dargestellten Weise einer der beiden miteinander in Eingriff kommenden Kontakte mit einer abgerundeten Stirnfläche ausgelegt, um keine unnötig hohe Genauigkeit bezüglich der Planparallelität bei Mehrfachkontakten einhalten zu müssen. Die erforderliche Kontaktkraft hängt vom metallischen Werkstoff und der benötigten Kontaktfläche (Stromtragfähigkeit) ab. Limitierend ist die Elastizitätsgrenze des elastischen Werkstoffes bei gegebener Auflagefläche des Kontaktes, die erheblich größer als die eigentliche Kontaktfläche sein muß.

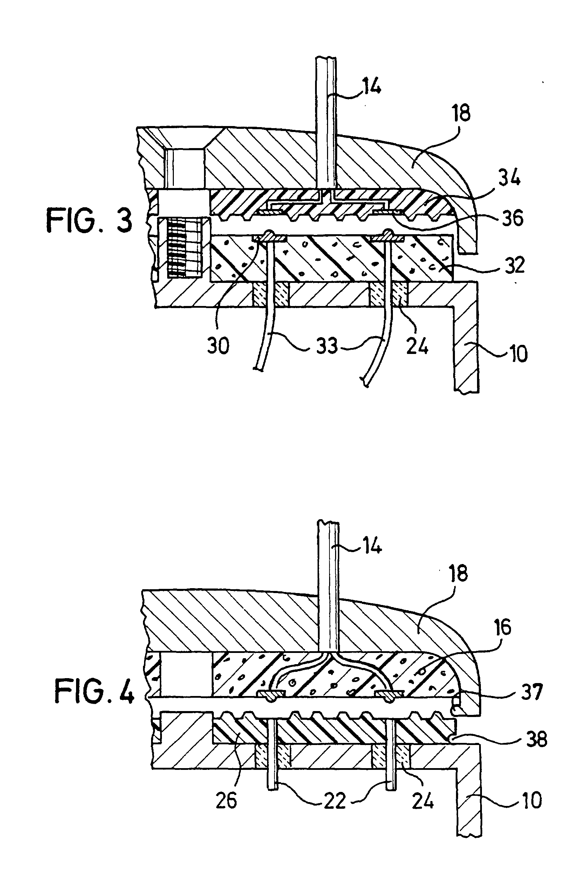

Die räumliche Anordnung von mit Dichtungsstegen versehenem starren Formkörper und elastischem Körper kann falls erwünscht vertauscht sein. FIG. 3 zeigt einen Ausschnitt eines Gehäuses 10 und einer Endkappe 18, wobei in diesem Fall auf die Stirnseite des Gehäuses 10, z.B. mittels Klebung, ein elastischer Körper 32 aufgebracht ist in welchen zwei Kontakte 30 mit näherungsweise halbkugelig abgerundeter Stirnfläche eingebettet sind. Die Kontakte 30 sind über Anschlußdrähte 33, die mittels Isolatoren 24 gegen das Gehäuse 10 abgeschirmt sind, mit der innerhalb des Gehäuses 10 untergebrachten elektrischen oder elektronischen Vorrichtung verbunden. Auf das Ende des Anschlußkabels 14 ist gemäß dieser Ausführungsform ein starres Anschlußstück 34 aufgebracht, welches analog dem in den FIG. 1 und 2 gezeigten Formkörper 26 mit Dichtungsstegen versehen ist und in welches gegenüberliegend den Kontakten 30 zwei scheibenförmige Kontakte 36 mit flacher Stirnfläche eingebettet sind.

Um Verwechslungen der Anschlußkabel während der Implantation der Einheit zu vermeiden, sind die Anschlußstücke vorzugsweise codiert oder sie unterscheiden sich in Form und/oder Größe. Eine weitere Möglichkeit der Codierung besteht darin, das eine der beiden Anschlußkabel, z.B. das zu einem Aktor führende Kabel, mit einem Anschlußstück gemäß FIG. 1 zu versehen, während für das andere Anschlußkabel, z.B. das zu einem Sensor führende Kabel, ein Anschlußstück gemäß FIG. 3 vorgesehen wird. Es versteht sich, daß die Stirnseite des Gehäuses 10 dann entsprechend mit einem Formkörper 26 und einem elastischen Körper 32 für die jeweiligen Anschlußstücke versehen wird.

Unabhängig davon, ob die Stirnseite des Gehäuses mit starren und/oder elastischen Körpern versehen wird, kann die Endkappe auch dauerhaft mit einem oder mehreren Anschlußstücken und Kabeln verbunden werden. Da in diesem Fall jedoch nicht das Auswechseln z.B. nur eines der Anschlußkabel möglich wäre, wird eine solche Ausführungsform weniger bevorzugt.

In FIG. 4 ist ein Ausschnitt ähnlich FIG. 3 einer weiter abgewandelten Ausführungsform der implantierbaren Einheit dargestellt, bei welcher der Eingriff zwischen dem Gehäuse und Verschlußkappe statt über eine Schraubverbindung über eine Rastverbindung erfolgt. Zu diesem Zweck weist die Endkappe 18 an der Innenseite ihres dem Gehäuse 10 zugewandten Randes einen oder mehrere Rastvorsprünge 37 auf, die beim Zusammenfügen von Gehäuse und Endkappe in entsprechende dem Gehäuse zugeordnete Ausnehmungen 38 einrasten. Bei der in FIG. 4 gezeigten Ausführungsform wird die Ausnehmung 38 von einem überstehenden Rand des auf die die Durchführungen 22 enthaltenden Seite des Gehäuses 10 aufgebrachten Formkörpers 26 gebildet. Es versteht sich, daß eine funktionsmäßig analoge Rastverbindung zwischen Endkappe und Gehäuse unabhängig von der Formgebung des Formkörpers 26 durch entsprechende Ausgestaltung der Endkappe 18 und des Gehäuses 10 selbst erreicht werden kann. Um die Kontaktkraft ***F***_{***K***} und die Dichtungskraft ***F***_{***D***} auch unter Verwendung einer Rastverbindung variieren zu können, können eine oder mehrere weitere Ausnehmungen parallel zu der Ausnehmung 38 vorgesehen sein, bzw. kann das mit dem Rastvorsprung in Eingriff tretende Bauteil eine sägezahnartige Oberfläche aufweisen.

Die grundlegenden funktionalen Größen des Verbindungsprinzips sind in den FIG. 5 und 6 aufgezeigt:
- Durchmesser der gasdichten Kontaktstelle Ø_{*K*}
   Der Durchmesser Ø_{*K*} muß so groß gewählt werden, daß beim ausgewählten metallischen Werkstoff die Stromtragfähigkeit gewährleistet ist.
- Durchmesser des elastisch gelagerten Kontaktes Ø_{*S*}
   Der Durchmesser Ø_{*S*} muß so groß gewählt werden, daß der elastische, elektrisch isolierende Werkstoff, der vorzugsweise aus Silikon besteht, nicht beschädigt wird bzw. beim Erzeugen der Rückstellkraft nicht über seine Elastizitätsgrenze hinaus beansprucht wird.
- Verformungsweg ***s***
   Wenn das Anschlußstück an dem auf der Stirnseite des Gehäuses angeordneten Körper anliegt, werden die Dichtungsstege durch den Verschlußmechanismus um den Verformungsweg ***s*** in den Formkörper gedrückt. Die resultierende Rückstellkraft erzeugt die Dichtungskraft ***F***_{***D***} und die Kontaktkraft ***F***_{***K***}. Shorehärte ***X*** und Elastizitätsgrenze des elastischen Werkstoffes spielen hierbei eine Rolle.
- Shorehärte ***X***
   Je höher die Shorehärte des elastischen Werkstoffes, desto höher ist die Rückstellkraft bei gegebenem Verformungsweg.
- Überstehende Höhe des Kontaktes ***h***_{***K***}
   Die mit dem Verschlußmechanismus erzeugte Kraft erzeugt sowohl den Druck auf den Dichtungsstegen als auch auf den Kontaktstellen. Werden die überstehende Höhe des Kontaktes ***h***_{***K***} und die Höhe der Dichtungsstege ***h***_{***D***} gleich gewählt, so verteilt sich der Druck proportional zu den jeweiligen Flächen, der durch die Federwirkung des elastischen Werkstoffes unter dem Verformungsweg ***s*** erzeugt wird. Wird die überstehende Höhe des Kontaktes ***h***_{***K***} anders als die Höhe der Dichtungsstege ***h***_{***D***} gewählt, so wird der volle Verformungsweg s nur bei einem der beiden Kontakte wirksam. Auf diese Weise kann der zu erzeugende Druck auf der Kontaktstelle und der zu erzeugende Druck auf den Dichtungsstegen unabhängig von deren Flächenverhältnissen eingestellt werden.
- Höhe der Dichtungsstege ***h***_{***D***}
   Es gilt der oben beschriebene Zusammenhang zur überstehenden Höhe des Kontaktes. Die erforderliche Höhe der Dichtungsstege hängt von der gesamten Geometrie der Dichtungsstege, des elastischen Werkstoffes und seiner Shorehärte ab. Wenn maximaler Druck auf den Dichtungsstegen herrschen soll, darf der elastische Werkstoff nicht an den Formkörper zwischen den Dichtungsstegen gepreßt werden. Die Höhe muß entsprechend gewählt werden und entspricht maximal dem Verformungsweg ***s*.**
- Breite der Dichtungsstege ***b***_{***D***}
   Je schmäler die Dichtungsstege gewählt werden, desto höher ist der Druck, jedoch darf der elastische Werkstoff nicht beschädigt werden.

Bei einer Stromtragfähigkeit im mA-Bereich kann der Raumbedarf der Kontaktanordnungen im Vergleich zu den oben aufgeführten normierten Herzschrittmachersteckverbindungen wesentlich minimiert werden.

Der Verschlußmechanismus muß die Anschlußstücke mit einer definierten Verschlußkraft gegen das Gehäuse drücken, so daß ein gasdichter Kontaktverschluß entsteht und die Dichtwirkung gewährleistet ist. Bevor die Kraft auf den elastischen Werkstoff einwirkt, der über den Verformungsweg ***s*** eine federartige Rückstellkraft erzeugt, müssen die Anschlußstücke in der exakt richtigen Position sein. Eine einfache Lösung stellt das Einlegen der Anschlußstücke in die Endkappe dar, die mit Hilfe einer Schraube mit definiertem Drehmoment an das Gehäuse gezogen wird. Dieser Prozeß ist jedoch nur bedingt möglich, wenn das Gehäuse bereits in einem Knochenbett verankert ist, weil der Verschluß von vom erfolgt.

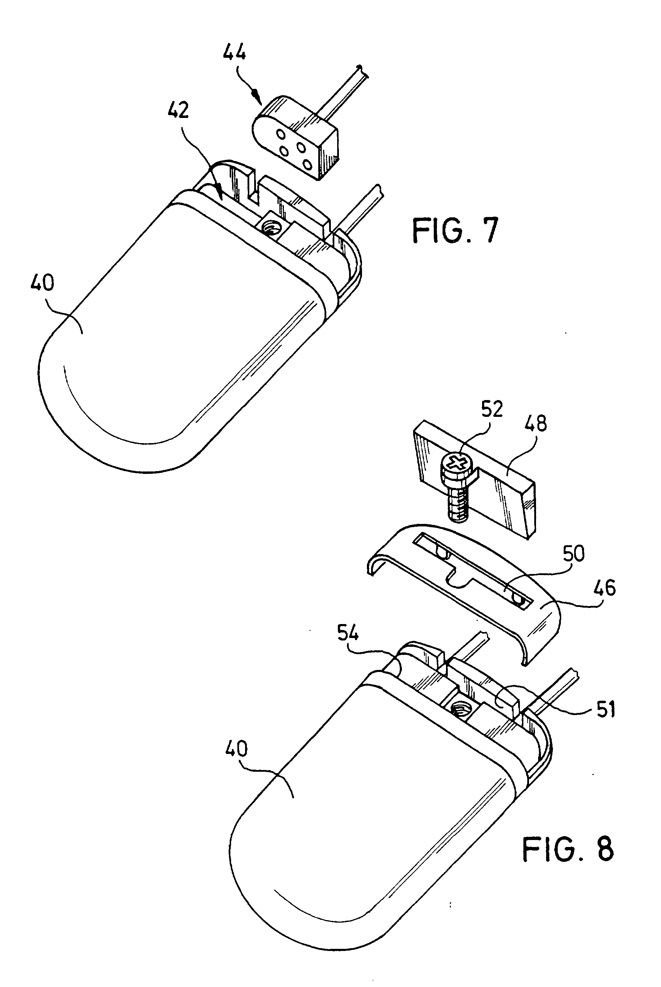

Um den Verschlußmechanismus von oben bedienen zu können, weist bei der in den FIG. 7 und 8 veranschaulichten abgewandelten Ausführungsform das perspektivisch gezeigte Gehäuse 40 an der Frontseite eine Art Wanne 42 auf, in welche Anschlußstücke 44 von oben eingebracht werden. Diese Anschlußstücke werden mit Hilfe einer im Bereich der Wanne 42 aufzusetzenden Abdeckung 46 in die exakte Position gebracht und dann über einen Keil 48 um einen definierten Verformungsweg gegen das Gehäuse 40 gedrückt. Die umlaufende Kontur der Anschlußstücke 44 und die entsprechende Aufnahme in der Wanne 42 können in der oben erwähnten Weise so gestaltet werden, daß kein Anschlußstück in die Aufnahme des anderen paßt. Ferner ist in FIG. 7 ein Anschlußstück 44 dargestellt, das für eine vierpolige Verbindung ausgelegt ist.

Entsprechend FIG. 8 wird nach dem Einsetzen der Anschlußstücke 44 in die Wanne 42 die Abdeckung 46 aufgesetzt. Durch eine in der Abdeckung vorgesehene Ausnehmung 50 wird ein Keil 48 zwischen die Anschlußstücke 44 und die dem Gehäuse 40 entfernt liegende Wandseite 51 der Wanne 42 eingebracht, so daß die Anschlußstücke 44 um den definierten Verformungsweg in Richtung auf die die Kontakte aufweisende Wandfläche 54 des Gehäuses 40 geschoben und gegen diese angepreßt werden. Dabei kann der Keil 48 mit Hilfe einer Schraube 52 eingebracht und fixiert werden, oder es kann ein (nicht gezeigter) Rastmechanismus vorgesehen sein, mittels dessen der manuell eingebrachte Keil 48 in der Einbaulage fixiert wird.

Das vorstehend beschriebene Verbindungsprinzip zwischen einer zu implantierenden Vorrichtung und aktorischen und/oder sensorischen Komponenten erlaubt eine Isolationsimpedanz zwischen den einzelnen Kontakten von mehr als 50 MΩ, wobei dieser Wert die von der oben erwähnten DIN-Norm für Herzschrittmacher geforderte Isolationsimpedanz von 50 kΩ um ein Vielfaches übersteigt. Zum Beispiel sind bei einem implantierbaren Hörgerät Verstärkungen von > 80 dB zwischen Sensor und Aktor erforderlich, ohne daß eine Rückkopplung zwischen den Sensorsignalen und den Aktorsignalen auftritt, und es sollten über die Kontaktsanordnung analoge Signale im µV-Bereich verzerrungsfrei übertragen werden können. Die vorgeschlagene implantierbare Einheit erlaubt es somit, die für implantierbare Hörgeräte geltenden hohen Ansprüche an die Art der Kontaktgebung zu befriedigen, die den Anforderungen der Signalübertragung im Audio- und HiFi-Bereich entsprechen.

Trotz eines hohen möglichen Miniaturisierungsgrades erfolgt das Schließen der Kontaktanordnungen einfach, sicher und zuverlässig. Ohne die Verwendung der typischen männlichen und weiblichen Ausbildung der Kontakte in Form eines Stiftes und einer zugehörigen Buchse besteht bei den vorgeschlagenen Systemen keine Gefahr, daß die Kontakte abgebrochen, verbogen oder sonstwie beschädigt werden können.

## Patentansprüche

1. Implantierbare Einheit mit mindestens einer Kontaktanordnung zur Verbindung einer in einem Gehäuse (10; 40) hermetisch dicht untergebrachten elektrischen oder elektronischen Vorrichtung (11) mit mindestens einem aus dem Gehäuse herausgeführten Anschlusskabel (12, 14), wobei die Kontaktanordnung einen ersten Kontakt (22; 36), einen auf einem elastischen Körper (16; 32) gelagerten zweiten Kontakt (30), einen Verschlussmechanismus (18, 19, 20; 48, 52) zum Ineingriffbringen der Stirnfläche des ersten Kontakts mit der Stirnfläche des zweiten Kontakts sowie mindestens einen den ersten Kontakt umgebenden Dichtungssteg (28) aufweist, der bei einem Eingriff der Kontakte in den elastischen Körper eingepresst ist und die Kontakte nach außen hin abdichtet, **dadurch gekennzeichnet, dass** der Dichtungssteg aus der den ersten Kontakt enthaltenden Ebene herausragt.

2. Implantierbare Einheit nach Anspruch 1, **dadurch gekennzeichnet, daß** eine Mehrzahl von zueinander konzentrischen Dichtungsstegen (28) vorgesehen ist.

3. Implantierbare Einheit nach Anspruch 1 oder 2, **dadurch gekennzeichnet, daß** eine Mehrzahl von Kontaktanordnungen (22, 30; 36) vorgesehen ist, und daß für jede Kontaktanordnung mindestens ein dieser Kontaktanordnung zugeordneter Dichtungssteg (28) vorgesehen ist.

4. Implantierbare Einheit nach einem der vorhergehenden Ansprüche, **dadurch gekennzeichnet, daß** der erste Kontakt (22) gehäusefest fixiert ist und mit der in dem Gehäuse (10) untergebrachten elektrischen oder elektronischen Vorrichtung (11) in Verbindung steht und daß der zweite Kontakt (30) mit dem Anschlußkabel (12, 14) in Verbindung steht.

5. Implantierbare Einheit nach Anspruch 4, **dadurch gekennzeichnet, daß** der erste Kontakt von einer Gehäusedurchführung (22) der elektrischen oder elektronischen Vorrichtung (11) gebildet ist und daß ferner ein elektrisch isolierender Formkörper (24) vorgesehen ist, welcher die Gehäusedurchführung umgibt.

6. Implantierbare Einheit nach einem der Ansprüche 1 bis 3, **dadurch gekennzeichnet, daß** der zweite Kontakt (30) dem Gehäuse (10) zugeordnet ist und mit der in dem Gehäuse untergebrachten elektrischen oder elektronischen Vorrichtung (11) in Verbindung steht und daß der erste Kontakt (36) mit dem mindestens einen Anschlußkabel (12, 14) in Verbindung steht.

7. Implantierbare Einheit nach einem der vorhergehenden Ansprüche, **dadurch gekennzeichnet, daß** der Verschlußmechanismus eine mit dem mindestens einen Anschlußkabel (12, 14) verbundene Verschlußkappe (18) aufweist, in welcher mindestens ein erster Kontakt (36) und/oder mindestens ein zweiter Kontakt (30) angeordnet ist, und daß eine Eingriffsanordnung (19, 20; 37, 38) vorgesehen ist, um die Verschlußkappe mit dem Gehäuse (10) in Eingriff halten.

8. Implantierbare Einheit nach einem der Ansprüche 1 bis 6, **dadurch gekennzeichnet, daß** an dem mindestens einen Anschlußkabel (12, 14) ein Anschlußstück (16, 34) vorgesehen ist, das einen oder mehrere erste oder zweite Kontakte (30, 36) trägt, daß der Verschlußmechanismus eine Verschlußkappe (18) mit einer Aufnahme für das mindestens eine Anschlußstück aufweist, und daß eine Eingriffsanordnung (19, 20; 37, 38) vorgesehen ist, um die Verschlußkappe mit dem Gehäuse (10) in Eingriff halten.

9. Implantierbare Einheit nach Anspruch 7 oder 8, **dadurch gekennzeichnet, daß** die Eingriffsanoränung mindestens ein dem Gehäuse (10) zugeordnetes Gewinde (19) sowie mindestens eine der Verschlußkappe (18) zugeordnete Schraube (20) aufweist.

10. Implantierbare Einheit nach Anspruch 9, **dadurch gekennzeichnet, daß** die Anordnung so getroffen ist, daß für einen Kontakt zwischen den ersten und zweiten Kontakten (22, 30; 36) ein definiertes Drehmoment, welches auf die Schraube (20) aufgebracht wird, erforderlich ist.

11. Implantierbare Einheit nach Anspruch 7 oder 8, **dadurch gekennzeichnet, daß** zur Ausbildung der Eingriffsanordnung eine zwischen der Verschlußkappe (18) und dem Gehäuse (10) wirkende Rastverbindung (37, 38) vorgesehen ist.

12. Implantierbare Einheit nach einem der Ansprüche 1 bis 6, **dadurch gekennzeichnet, daß** an dem mindestens einen Anschlußkabel (12, 14) ein Anschlußstück (16, 34) vorgesehen ist, das Gehäuse (40) eine wannenförmige Verlängerung (42) zur Aufnahme des mindestens einen Anschlußstücks aufweist, und ferner ein Keil (48) zum Einbringen zwischen die dem Gehäuse entfernt liegende Wandseite (51) der wannenförmigen Verlängerung und ein in die wannenförmige Verlängerung eingelegtes Anschlußstück vorgesehen ist.

13. Implantierbare Einheit nach Anspruch 12, **dadurch gekennzeichnet, daß** zum Einbringen und Fixieren des Keils (48) mindestens eine Schraube (52) vorgesehen ist.

14. Implantierbare Einheit nach einem der vorhergehenden Ansprüche, **dadurch gekennzeichnet, daß** der elastische Körper (16, 32) aus Silikon besteht.

15. Implantierbare Einheit nach einem der vorhergehenden Ansprüche, **dadurch gekennzeichnet, daß** jeweils einer der ersten (22, 36) oder zweiten (30) Kontakte eine abgerundete Oberfläche aufweist.

16. Implantierbare Einheit nach Anspruch 15, **dadurch gekennzeichnet, daß** die mit der abgerundeten Oberfläche eines ersten (16, 32) oder zweiten (30) Kontakts in Eingriff tretende Oberfläche des zugehörigen zweiten bzw. ersten Kontakts näherungsweise eben ist.

## Claims

1. Implantable unit comprising at least one contact arrangement for connection of an electrical or electronic device (11) which is hermetically sealed within a housing (10; 40) to at least one connection cable (12, 14) which is routed out of the housing, wherein the contact arrangement has a first contact (22; 36), a second contact (30) supported on an elastic body (16; 32), a closing mechanism (18, 19, 20; 48, 52) for engaging the face of the first contact with the face of the second contact, as well as at least one sealing land (28) which surrounds the first contact, and which upon engagement of the contacts is pressed into the elastic body and seals the contacts relative to the exterior, **characterized in that** the sealing land protrudes from the plane containing the first contact.

2. Implantable unit according to claim 1, **characterized in that** there is provided a plurality of concentric sealing lands (28).

3. Implantable unit according to claim 1 or 2, **characterized in that** there is provided a plurality of contact arrangements (22, 30; 36), and **in that** for each of said contact arrangements there is provided at least one sealing land (28) which is associated to such contact arrangement.

4. Implantable unit according to any one of the preceding claims, **characterized in that** the first contact (22) is fixed with respect to the housing and is connected to the electrical or electronic device (11) housed with the housing (10), and **in that** the second contact (30) is connected to the connection cable (12, 14).

5. Implantable unit according to claim 4, **characterized in that** the first contact is formed by a housing feedthrough (22) of the electrical or electronic device (11), and **in that** further an electrically insulating formed body (24) surrounds the housing feedthrough.

6. Implantable unit according to any one of claims 1 to 3, **characterized in that** the second contact (30) is associated with the housing (10) and is connected to the electrical or electronic device (11) housed within the housing, and **in that** the first contact (36) is connected to the at least one connection cable (12, 14).

7. Implantable unit according to any one of the preceding claims, **characterized in that** the closing mechanism comprises a closing cap (18) which is connected to the at least one connection cable (12, 14) and in which at least one first contact (36) and/or at least one second contact (30) is located, and **in that** an engagement arrangement (19, 20; 37, 38) is provided for maintaining the closing cap in engagement to the housing (10).

8. Implantable unit according to any one of claims 1 to 6, **characterized in that** a terminal fitting (16, 34) is provided on said at least one connection cable (12, 14), which terminal fitting carries one or more first or second contacts (30, 36), that the closing mechanism has a closing cap (18) with a receiver for the at least one terminal fitting; and that an engagement arrangement (19, 20; 37, 38) is provided for maintaining the closing cap in engagement to the housing (10).

9. Implantable unit according to claim 7 or 8, **characterized in that** the engagement arrangement comprises at least one thread (19) associated to the housing (10) and at least one screw (20) associated to the closing cap (18).

10. Implantable unit according to claim 9, **characterized in that** it is designed such that a defined torque acting on the screw (20) is required to provide for a contact between the first and second contacts (22, 30, 36).

11. Implantable unit according to claim 7 or 8, **characterized in that** a catch connection (37, 38) which acts between the closing cap (18) and the housing (10) is provided to form the engagement arrangement.

12. Implantable unit according to any one of claims 1 to 6, **characterized in that** a terminal fitting (16, 34) is provided on said at least one connection cable (12, 14), that the housing (40) has a trough-shaped extension (42) for holding the at least one terminal fitting; and that a wedge (48) to be inserted between the wall (51) of the trough-shaped extension remote from the housing and a terminal fitting inserted into the trough-shaped extension is provided.

13. Implantable unit according to claim 12, **characterized in that** at least one screw (52) is provided for inserting and fixing the wedge (48).

14. Implantable unit according to any one of the preceding claims, **characterized in that** the elastic body (16, 32) is made of silicone.

15. Implantable unit according to any one of the preceding claims, **characterized in that** one of the first (22, 36) or second (30) contacts has a rounded engagement surface.

16. Implantable unit according to claim 15, **characterized in that** the surface of the respective second or first contact, which is engaged by said rounded surface of the first (16, 32) or second (30) contact, respectively, has an approximately flat surface.

## Revendications

1. Unité implantable comportant au moins un agencement de contact destiné au raccordement d'un dispositif (11) électrique ou électronique logé de manière hermétique et étanche dans un boîtier (10 ; 40) comportant au moins un câble de raccordement (12, 14) conduit hors du boîtier, l'agencement de contact présentant un premier contact (22 ; 36), un second contact (30) logé sur un corps élastique (16 ; 32), un mécanisme de fermeture (18, 19, 20 ; 48, 52) en vue de la mise en prise de la face terminale du premier contact avec la face terminale du second contact ainsi qu'au moins une entretoise d'étanchéité (28) entourant le premier contact qui est enfoncée dans le corps élastique lors d'une mise en prise des contacts et qui étanchéifie les contacts envers l'extérieur, **caractérisée en ce que** l'entretoise d'étanchéité dépasse du plan contenant le premier contact.

2. Unité implantable selon la revendication 1, **caractérisée en ce qu'**une pluralité d'entretoises d'étanchéité (28) concentriques les unes par rapport aux autres est prévue.

3. Unité implantable selon la revendication 1 ou 2, **caractérisée en ce qu'**une pluralité d'agencements de contact (22, 30 ; 36) est prévue, et **en ce que** pour chaque agencement de contact, au moins une entretoise d'étanchéité (28) affectée à cet agencement de contact est prévue.

4. Unité implantable selon l'une quelconque des revendications précédentes, **caractérisée en ce que** le premier contact (22) est fixé solidairement au boîtier et est raccordé au dispositif (11) électrique ou électronique logé dans le boîtier (10) et **en ce que** le second contact (30) est raccordé au câble de raccordement (12, 14).

5. Unité implantable selon la revendication 4, **caractérisée en ce que** le premier contact est formé par une traversée du boîtier (22) du dispositif (11) électrique ou électronique et **en ce qu'**en outre, un corps moulé (24) isolant électrique est prévu, lequel entoure la traversée du boîtier.

6. Unité implantable selon l'une quelconque des revendications 1 à 3, **caractérisée en ce que** le second contact (30) est affecté au boîtier (10) et est raccordé au dispositif (11) électrique ou électronique logé dans le boîtier et **en ce que** le premier contact (36) est raccordé au câble de raccordement (12, 14) au nombre minimum d'un.

7. Unité implantable selon l'une quelconque des revendications précédentes, **caractérisée en ce que** le mécanisme de fermeture présente un chapeau d'obturation (18) raccordé au câble de raccordement (12, 14) au nombre minimum d'un, au moins un premier contact (36) et/ou au moins un second contact (30) étant disposé dans ledit chapeau d'obturation, et **en ce qu'**un agencement de prise (19, 20 ; 37, 38) est prévu afin de maintenir le chapeau d'obturation en prise avec le boîtier (10).

8. Unité implantable selon l'une quelconque des revendications 1 à 6, **caractérisée en ce qu'**une pièce de raccordement (16, 34) est prévue au niveau de l'au moins un câble de raccordement (12, 14), ladite pièce de raccordement portant un ou plusieurs premiers et seconds contacts (30, 36), **en ce que** le mécanisme de fermeture présente un chapeau d'obturation (18) avec un logement pour l'au moins une pièce de raccordement, et **en ce qu'**un agencement de prise (19, 20 ; 37, 38) est prévu afin de maintenir le chapeau d'obturation en prise avec le boîtier (10).

9. Unité implantable selon la revendication 7 ou 8, **caractérisée en ce que** l'agencement de prise présente au moins un filetage (19) affecté au boîtier (10) ainsi qu'au moins une vis (20) affectée au chapeau d'obturation (18).

10. Unité implantable selon la revendication 9, **caractérisée en ce que** l'agencement est réalisé de manière à ce qu'un couple de rotation défini, lequel est appliqué sur la vis (20), est nécessaire pour un contact entre les premiers et seconds contacts (22, 30 ; 36).

11. Unité implantable selon la revendication 7 ou 8, **caractérisée en ce qu'**un assemblage à crans (37, 38) opérant entre le chapeau d'obturation (18) et le boîtier (10) est prévu en vue de la réalisation de l'agencement de prise.

12. Unité implantable selon l'une quelconque des revendications 1 à 6, **caractérisée en ce qu'**une pièce de raccordement (16, 34) est prévue au niveau de l'au moins un câble de raccordement (12, 14), **en ce que** le boîtier (40) présente un prolongement en forme de cuve (42) destiné au logement de l'au moins une pièce de raccordement, et **en ce qu'**en outre, une cale (48) est prévue en vue de l'insertion entre le côté paroi (51) du prolongement en forme de cuve se trouvant distant du boîtier et une pièce de raccordement insérée dans le prolongement en forme de cuve.

13. Unité implantable selon la revendication 12, **caractérisée en ce qu'**au moins une vis (52) est prévue en vue de l'insertion et de la fixation de la cale (48).

14. Unité implantable selon l'une quelconque des revendications précédentes, **caractérisée en ce que** le corps élastique (16, 32) est en silicone.

15. Unité implantable selon l'une quelconque des revendications précédentes, **caractérisée en ce que** chacun des premiers (22, 36) ou des seconds (30) contacts présente une surface arrondie.

16. Unité implantable selon la revendication 15, **caractérisée en ce que** la surface du second ou du premier contact correspondant qui entre en contact avec la surface arrondie d'un premier (16, 32) ou d'un second (30) contact est approximativement plane.
